# DEMANDE DE BREVET EUROPEEN

(11) **EP 2 540 335 A1**
(43) Date de publication de la demande: **02.01.2013**
(21) Numéro de dépôt: 11005292.5
(22) Date de dépôt: 29.06.2011
(51) Int. Cl.: A61M 16/16, A61M 16/00, A61M 16/10

(54) **Reservoir pour appareil d'assistance respiratoire**

(71) Demandeur: HEALTHC'AIR, 54600 Villers Les Nancy (FR)
(72) Inventeur: Perine, Philippe, 54690 Eulmont (FR); Poirot, Bertrand, 54600 Villiers-Les-Nancy (FR); Mocellin, Arnaud, 54200 Choloy Menillot (FR)
(74) Mandataire: Novagraaf Technologies

(57) **Abrégé**

Réservoir, pour un appareil d'assistance respiratoire, comprenant un bac (4) pour contenir un fluide destiné à être entraîné par le flux gazeux, un couvercle (1), et un élément d'étanchéité (2) entre le bac (4) et le couvercle 1. L'élément d'étanchéité (2) comprend notamment un joint périphérique (22) et une entretoise interne (21) assurant le positionnement respectif d'un embout d'arrivée (31) du flux gazeux et d'un embout de sortie (32) du flux gazeux chargé en fluide.

## Description

La présente invention concerne un réservoir apte à contenir un fluide pour l'enrichissement d'un gaz à délivrer à un patient par un appareil d'assistance respiratoire, par exemple un réservoir à eau pour un appareil de pression positive pour le traitement des troubles respiratoires du sommeil.

Un patient souffrant, par exemple, d'un syndrome d'apnée du sommeil est généralement traité par un appareil à pression positive délivrant un flux d'air et associé à un humidificateur, qui consiste en un réservoir à eau positionné sur une plaque chauffante, afin d'éviter l'assèchement des voies respiratoires du patient. Le flux d'air généré par l'appareil traverse alors le réservoir à eau avant d'arriver dans un conduit pour ventiler le patient.

Dans ce domaine, on connait par exemple des réservoirs dont la sortie d'air est horizontale, ce qui favorise des risques de pénétration de fluide dans l'appareil, par exemple, suite à de mauvaises manipulations.

Ces risques de pénétration de fluide dans l'appareil peuvent alors entraîner un endommagement de l'appareil et de ses composants internes.

Ainsi, par mesures de sécurité, il est préférable que l'appareil ne puisse pas être utilisé par le patient si tous les composants du système d'humidification, et notamment des éléments d'étanchéité, ne sont pas assemblés, tout en limitant les risques de reflux de fluide dans l'appareil.

Par ailleurs, il est aussi nécessaire que le flux gazeux circule suffisamment dans le réservoir pour se charger en fluide, et notamment en humidité.

L'invention vise à remédier aux inconvénients de l'état de la technique, et plus particulièrement, l'invention a pour but de proposer un réservoir permettant de prévenir des reflux de fluide dans l'appareil en optimisant l'écoulement du flux de gaz dans le réservoir pour que celui-ci se charge suffisamment en fluide. Enfin, la présente invention vise aussi un réservoir dont les éléments constitutifs peuvent être intégralement lavés pour amoindrir, voire éviter, tout problème d'hygiène dû au fait que certains éléments intérieurs à un réservoir sont souvent inaccessibles.

L'invention, selon un premier aspect, a ainsi pour objet un réservoir pour un appareil d'assistance respiratoire comprenant une turbine pour générer un flux gazeux, ledit réservoir comprenant un bac pour contenir un fluide destiné à être entrainé par le flux gazeux, un couvercle, et un élément d'étanchéité entre le bac et le couvercle, l'élément d'étanchéité comprenant un joint périphérique qui assure l'étanchéité à la liaison entre le bac et le couvercle, et une entretoise interne assurant le positionnement respectif d'un embout d'arrivée du flux gazeux et d'un embout de sortie du flux gazeux chargé en fluide.

Ainsi, un tel réservoir est complètement démontable ce qui permet de nettoyer facilement les différents composants, ce qui contribue à limiter les problèmes d'hygiène.

La géométrie de l'élément d'étanchéité, de préférence en une seule pièce, permet un positionnement précis des embouts, de sorte que l'écoulement du flux gazeux dans le réservoir est suffisant pour assurer la charge du gaz en fluide.

On entend par « suffisant » que le flux gazeux comprend, en sortie du réservoir, une teneur en fluide conforme aux prescriptions standard permettant au moins d'éviter l'assèchement des voies respiratoires du patient.

L'agencement des différents composants (couvercle, bac, élément d'étanchéité et embouts) permet ainsi non seulement de faire le lien entre une entrée du flux gazeux dans le réservoir et une sortie du flux gazeux du réservoir pour être délivré à un patient, mais aussi de limiter les risques de reflux du fluide dans l'appareil, ce qui risquerait de l'endommager.

En outre, la forme du réservoir, et donc de l'élément d'étanchéité peut contribuer à assurer que l'élément d'étanchéité n'est positionnable que d'une seule façon. Par exemple, une paroi du réservoir peut être courbe.

Le bon positionnement de l'élément d'étanchéité par rapport au couvercle et au bac peut aussi être indiqué par un code couleur ou la présence de rainures et de nervures, ou encore des variations de texture ce qui peut s'avérer très utile dans le cas d'une utilisation par une personne mal voyante.

Selon un mode préférentiel de réalisation, l'entretoise interne de l'élément d'étanchéité assure aussi la fixation de chacun des embouts.

La limitation du nombre de pièces constituant le réservoir permet aussi de simplifier son assemblage.

Les embouts peuvent par exemple être emmanchés dans des orifices prévus à cet effet.

L'utilisation d'un matériau polymère est donc avantageuse pour former l'élément d'étanchéité car un matériau polymère procure élasticité et adhérence.

L'entretoise servant à la fois au positionnement et au maintien des embouts sert ainsi de détrompeur pour assembler les embouts avec l'élément d'étanchéité.

Tout type de détrompage permettant d'assurer le bon positionnement des embouts (31, 32) peut convenir (par exemple : code couleur, diamètre des embouts (31, 32) différents en accord avec les orifices d'entrée et de sortie (11, 12) du couvercle, nervure-rainure, formes différentes).

Avantageusement, le couvercle du réservoir comprend un orifice d'entrée du flux gazeux apte à être relié à une bouche d'échappement de gaz de l'appareil, et un orifice de sortie du flux gazeux chargé en fluide apte à être connecté à un conduit pour délivrer le flux gazeux chargé en fluide à un patient.

Il n'est ainsi pas possible d'utiliser l'appareil tant que tous les éléments du réservoir ne sont pas correctement positionnés.

Par exemple, s'il manque le couvercle, il n'est pas possible d'assembler l'ensemble réservoir à l'appareil. S'il manque le bac, rien n'est positionnable. S'il manque l'élément d'étanchéité, il n'est plus possible de positionner les embouts et en outre l'orifice d'entrée du couvercle n'est plus apte à arriver en vis-à-vis de la bouche d'échappement de l'appareil. S'il manque l'embout, il n'est pas possible de relier un conduit pour délivrer le flux gazeux chargé en fluide au patient.

De plus, il est préférable que le joint périphérique soit nécessaire pour assembler le bac et le couvercle. C'est-à-dire que le bac et le couvercle ne présentent pas de forme complémentaire permettant de les assembler directement.

Selon un mode préférentiel de réalisation, le couvercle comprend une cloison apte à coopérer avec l'entretoise interne de l'élément d'étanchéité pour délimiter un circuit étanche, entre l'embout d'arrivée du flux gazeux et l'orifice d'entrée du réservoir, et/ou entre l'embout de sortie du flux gazeux et l'orifice de sortie du réservoir ce qui renforce l'étanchéité.

La présence d'un tel circuit étanche prolonge l'embout par l'assemblage de plusieurs éléments ce qui permet de limiter les zones difficiles d'accès pour nettoyer les éléments constitutifs du réservoir.

Avantageusement, l'entretoise interne présente une gorge apte à recevoir la cloison du couvercle pour délimiter le circuit étanche.

La coopération des différents éléments est nécessaire pour assurer l'étanchéité du parcours du flux de gaz. De plus, la présence de la gorge permet de garantir un bon positionnement des différents éléments.

Selon un mode préférentiel de réalisation, l'embout d'arrivée du flux gazeux se prolonge sous l'entretoise par un tronçon coudé puis par un tronçon rectiligne. Le tronçon coudé peut présenter un angle obtus, droit, aigu ou plat, de sorte que le tronçon rectiligne peut être orienté selon toute direction, être horizontal ou vertical.

La configuration de l'embout et des tronçons permet ainsi de garantir l'écoulement du flux de gaz dans le réservoir de sorte à ce qu'il se charge suffisamment en fluide. En effet, le gaz doit pouvoir demeurer suffisamment longtemps dans le réservoir pour se charger en fluide. L'embout prolongé par un tronçon coudé et un tronçon rectiligne oriente le flux et lui donne une vitesse nécessaire pour cela.

Avantageusement, le tronçon rectiligne de l'embout d'arrivée du flux gazeux débouche sensiblement au centre de gravité du réservoir, car un tel positionnement permet en outre de limiter tout reflux de fluide.

En effet, un tel positionnement permet de réduire le risque de pénétration de fluide dans l'appareil et/ou dans le circuit patient car le centre de gravité est la position la plus épargnée par l'eau lors de rotations du réservoir, quel que soit l'axe de rotation.

Selon un mode préférentiel de réalisation, l'embout de sortie du flux gazeux se prolonge au-dessus de l'entretoise par un tronçon rectiligne, de préférence vertical.

L'orientation verticale facilite la sortie du flux de gaz chargé en fluide, notamment à cause du fait que l'appareil de traitement est souvent posé au sol ou sur une table de chevet alors que le patient est alité, ce qui permet de limiter de potentielles tortuosités au conduit permettant de délivrer le gaz au patient et qui pourraient dans certains cas obstruer le conduit, par exemple si celui-ci plie au lieu de se courber.

De préférence, le tronçon rectiligne de l'embout de sortie du flux gazeux débouche à l'extérieur du couvercle du réservoir.

Ainsi, lorsqu'il est assemblé sur le couvercle du réservoir, le tronçon rectiligne de l'embout de sortie du flux gazeux est apte à former un tube de sortie de l'ensemble réservoir. Il permet alors la connexion avec le conduit du patient ou toute autre interface entre le réservoir et le patient.

L'étanchéité entre le couvercle et le tronçon rectiligne de l'embout de sortie peut être assurée par un joint torique par exemple, ou par un pas de vis qui permettrait d'assurer à la fois l'étanchéité et la fixation de l'embout de sortie.

Ceci contribue aussi à la sécurité d'utilisation induite par le fait que le réservoir n'est pas utilisable tant que tous les éléments ne sont pas correctement agencés. Par voie de conséquence, si le réservoir n'est pas utilisable, l'appareil équipé d'un tel réservoir ne l'est pas non plus.

Selon un mode préférentiel de réalisation, l'embout d'arrivée du flux gazeux est solidaire de l'embout de sortie du flux gazeux grâce à une bride de liaison, de sorte qu'ils ne forment qu'une pièce unique.

Ceci permet de faciliter davantage l'assemblage du réservoir. De plus, en coopération avec l'entretoise, la liaison entre les deux embouts renforce l'aspect détrompeur lors de l'assemblage. Toute personne cherchant à assembler le réservoir ne peut donc pas mal l'assembler : soit le réservoir est assemblé et utilisable, soit il n'est pas assemblé et les pièces ne tiennent pas ensemble et il n'est alors pas utilisable.

Avantageusement, la bride de liaison est disposée sous l'entretoise interne, ce qui permet d'assembler d'abord l'élément d'étanchéité avec le couvercle, puis ensuite avec les embouts.

Avantageusement, la pièce unique formée par l'embout d'arrivée du flux gazeux et l'embout de sortie du flux gazeux comporte un crochet de verrouillage de l'assemblage de la pièce unique et du couvercle, s'étendant de préférence verticalement, apte à coopérer avec un élément complémentaire porté par le couvercle.

Ceci permet non seulement le maintien en position des différentes éléments les uns par rapport aux autres, mais ceci permet en outre d'éviter que le poids des embouts supporté par l'entretoise de l'élément d'étanchéité ne flue.

Tout type d'élément permettant d'assurer le positionnement et la fixation des embouts, qu'ils forment ou non une pièce unique, par rapport au couvercle peut convenir (clippage, butée de positionnement, nervure, emmanchement, vissage, pièce tierce du type goupille...). Dans le cas où les embouts sont deux pièces séparées, chacun peut être muni de son propre élément de fixation, de même type que ceux énoncés précédemment .

Dans le cas où les embouts ne forment qu'une pièce unique, le positionnement du crochet de verrouillage assure à la fois le maintien en position et le détrompage car il n'est possible de fixer la pièce d'embouts au couvercle que d'une seule.

Avantageusement, la pièce unique formée par l'embout d'arrivée du flux gazeux et l'embout de sortie du flux gazeux comporte une première butée apte à coopérer avec un élément du couvercle, ce qui permet d'assurer la stabilité du positionnement des éléments entre eux. Selon un mode préférentiel de réalisation, le crochet de verrouillage et la première butée peuvent ne constituer qu'une seule pièce.

Avantageusement, la pièce unique formée par l'embout d'arrivée du flux gazeux et l'embout de sortie du flux gazeux comporte une autre butée apte à venir en appui sur un élément en sailli d'une paroi latérale du bac, ce qui permet à la fois un bon positionnement du bac par rapport aux autres éléments et évite que le joint périphérique soit cisaillé entre le bac et le couvercle lorsqu'ils sont assemblés. Cette butée peut en outre être bloquée latéralement par des cales, qui peuvent être elles aussi solidaires de l'élément en sailli de la paroi latérale du bac.

De préférence, le bac du réservoir comprend un fond, de préférence métallique, apte à être positionné sur une plaque chauffante de l'appareil. Le chargement du gaz en fluide est alors facilité du fait que le fluide contenu dans le réservoir peut être chauffé. Un fond métallique assure ainsi une meilleure conduction thermique sans risque de pollution du fluide contenu dans le bac.

Selon un mode avantageux de réalisation, le fond métallique peut être détaché du bac du réservoir, ce qui facilite par exemple un détartrage du fond ou un nettoyage particulier. En outre, il est préférable que le fond métallique soit par exemple réalisé en acier inoxydable, par exemple pour limiter les risques d'oxydation du métal. Il est aussi possible de réaliser le fond métallique, par exemple, en plastique chargé en acier, ce qui est un matériau procurant l'avantage d'une stabilité dimensionnelle, d'une industrialisation plus facile (à moindre coût), et pas de risque d'oxydation. Ou encore, le fond pourrait être constitué d'un matériau comprenant une base céramique, ce qui procure aussi une stabilité dimensionnelle, mais aussi un transfert thermique et stabilité thermique (ce qui permet de limiter la consommation d'énergie), une tenue dans le temps, et pas de risque d'oxydation.

Un réservoir tel que décrit précédemment présente donc l'avantage d'être facilement assemblé, par exemple de la façon suivante :
- On assemble l'embout d'arrivée du flux gazeux et l'embout de sortie du flux gazeux avec l'entretoise de l'élément d'étanchéité ;
- On fixe l'élément d'étanchéité assemblé avec les embouts sur le couvercle ;
- On assemble le couvercle et le bac.

Ou de la façon suivante :
- On fixe l'élément d'étanchéité sur le couvercle ;
- On positionne l'embout d'arrivée du flux gazeux et l'embout de sortie du flux gazeux sur l'élément d'étanchéité ;
- On fixe l'embout d'arrivée du flux gazeux et l'embout de sortie du flux gazeux sur le couvercle ;
- On assemble le couvercle et le bac.

Inversement, le réservoir est aisément démontable par exemple pour être nettoyé, de la façon suivante :
- On retire le couvercle du bac ;
- On décroche les embouts du couvercle ;
- On retire l'élément d'étanchéité du couvercle ;
- On sépare les embouts de l'entretoise interne de l'élément d'étanchéité.

Bien sûr, ces différentes étapes peuvent être réalisées sans ordre préférentiel à la guise de l'utilisateur.

La présente invention a aussi pour objet un appareil d'assistance respiratoire comprenant un réservoir tel que décrit précédemment.

Selon un mode préférentiel de réalisation, l'appareil comprend en outre un connecteur souple, avantageusement proéminent, fixé sur la bouche d'échappement de gaz de l'appareil, de sorte que lorsque le réservoir est en place, le connecteur est en compression et assure l'étanchéité entre la bouche d'échappement de gaz de l'appareil et l'orifice d'arrivée du flux gazeux du réservoir.

Enfin, la présente invention vise l'utilisation d'un appareil tel que décrit précédemment pour le traitement des troubles respiratoires du sommeil, et notamment pour le traitement de l'apnée du sommeil.

L'invention, selon un mode préférentiel de réalisation, sera bien comprise et ses avantages apparaîtront mieux à la lecture de la description détaillée qui suit, à titre indicatif et nullement limitatif, et en référence aux dessins annexés présentés ci-après :
- La figure 1, est un schéma fonctionnel de l'invention.
- La figure 2 représente une vue en coupe de profil d'un réservoir selon un mode de réalisation préférentiel de l'invention.
- La figure 3 représente une vue en coupe de dessus du réservoir.
- La figure 4 représente une vue en coupe en perspective du réservoir.
- La figure 5 représente une vue en coupe en perspective du couvercle, de l'élément d'étanchéité et des embouts assemblés, selon le mode préférentiel de réalisation de l'invention.
- La figure 6 représente une vue selon deux coupes en perspective de l'assemblage du couvercle, de l'élément d'étanchéité, des embouts, et du bac pour contenir un fluide, selon le mode préférentiel de réalisation de l'invention.
- La figure 7 représente la figure 6 sans le bac pour contenir un fluide.
- La figure 8 représente un éclaté du réservoir.

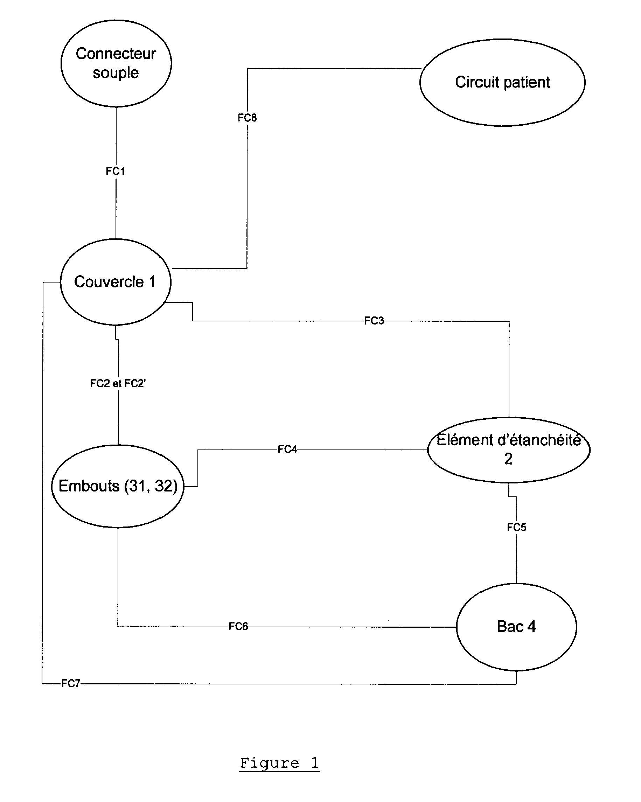

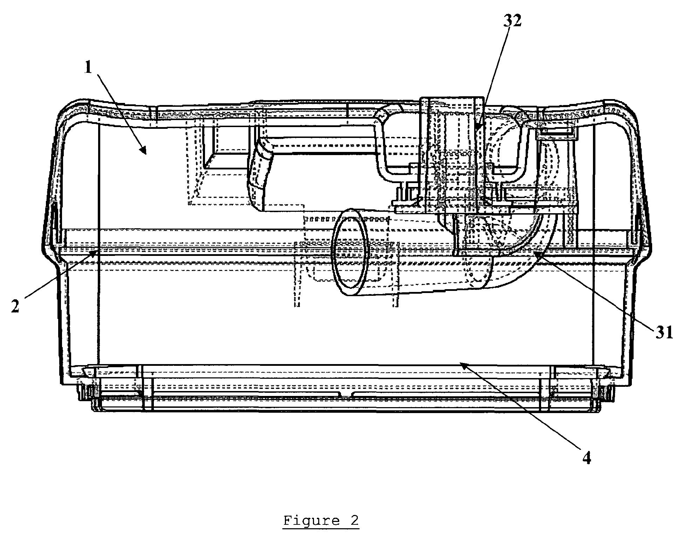

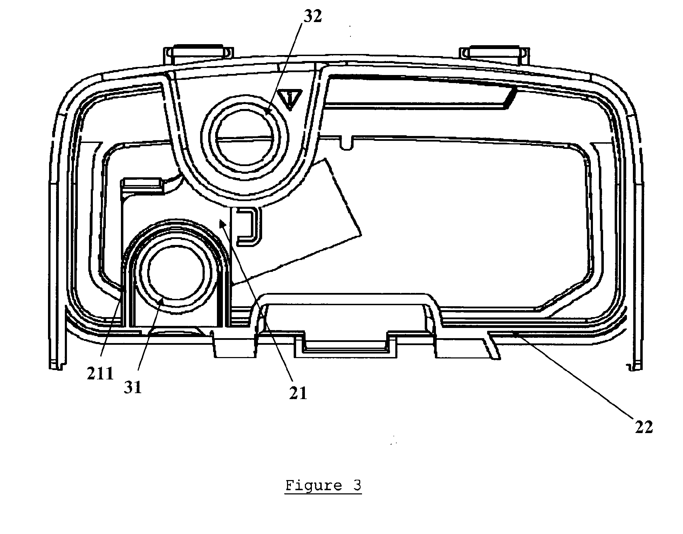

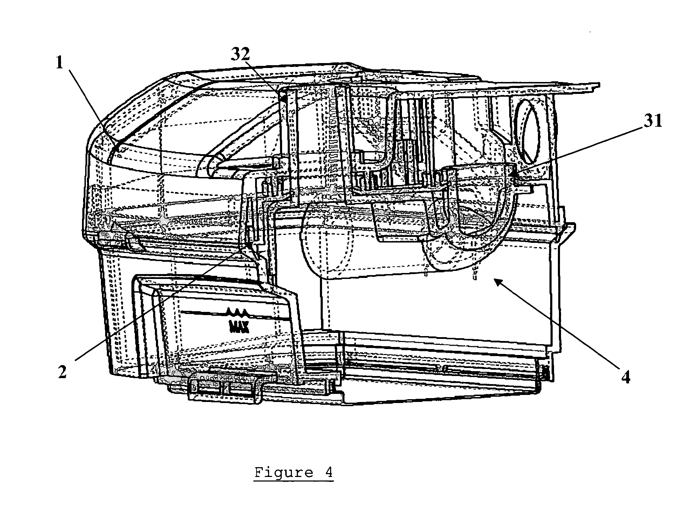

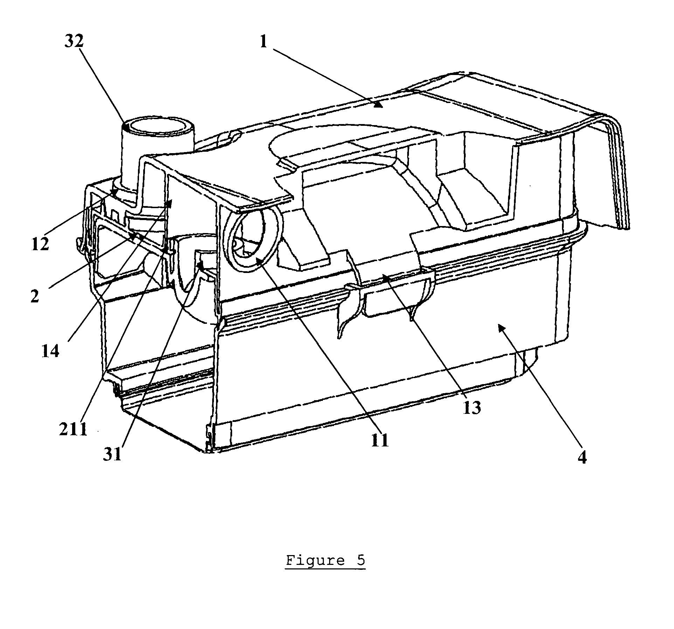

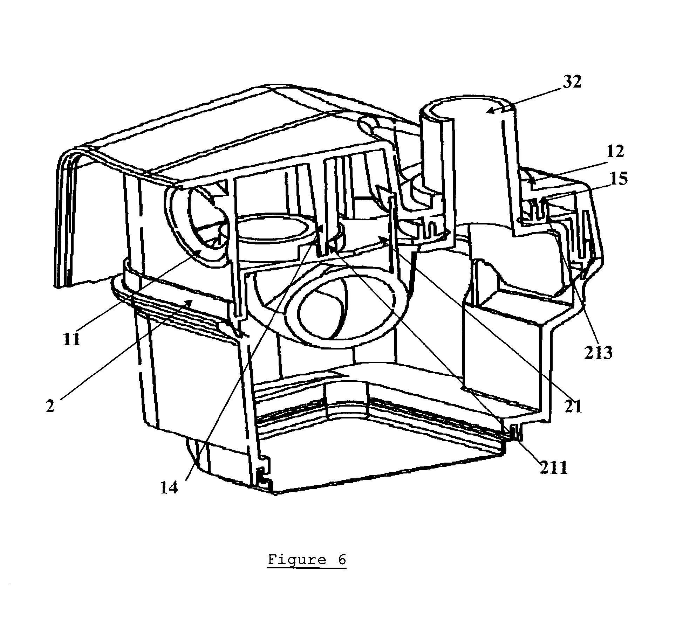

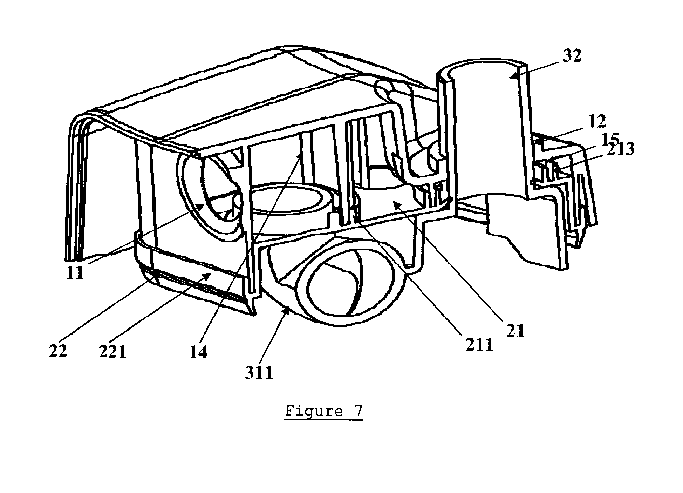

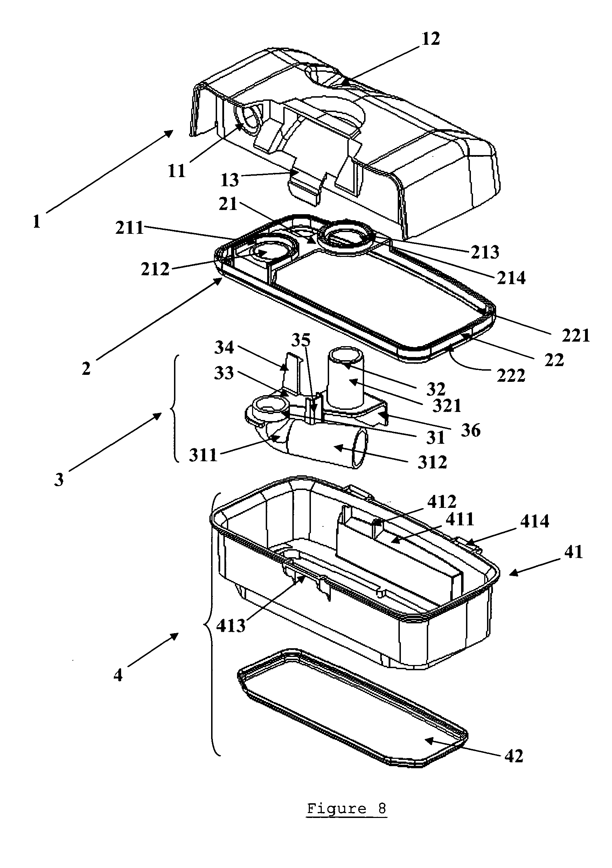

Les éléments identiques représentés sur les figures 1 à 8 sont identifiés par des références numériques identiques.

Un réservoir selon l'invention comprend un couvercle 1, un élément d'étanchéité 2, un embout d'arrivée d'air 31 et un embout de sortie d'air 32, et un bac 4 pour contenir un fluide.

Le schéma fonctionnel de la figure 1 permet d'expliciter les différentes interactions existant entre les pièces constituant le réservoir.

FC1 : Le couvercle 1 est directement en contact avec un connecteur souple d'un appareil pour assurer l'étanchéité entre le couvercle 1 et une bouche d'échappement de gaz de l'appareil. Avantageusement, le connecteur est donc en compression lorsque le réservoir est assemblé et mis en place sur l'appareil.

FC2 et FC2' : Le couvercle 1 est relié aux embouts (31, 32) par au moins un crochet apte à coopérer avec un élément complémentaire porté par le couvercle 1. Dans le présent exemple de réalisation, les embouts (31, 32) sont reliés par une bride de liaison 33 pour former une pièce unique 3. La bride de liaison 33 présente un crochet de verrouillage 34, ainsi que différentes butées (35, 36) permettant d'assurer son positionnement et sa stabilité par rapport à l'ensemble du réservoir. Le couvercle 1 est relié à l'embout d'arrivée de flux gazeux 31 par l'intermédiaire d'une entretoise interne de l'élément d'étanchéité 2. L'embout d'arrivée du flux gazeux 31 traverse l'entretoise interne de l'élément d'étanchéité 2 par un orifice 212, et l'embout de sortie du flux gazeux 32 traverse l'entretoise interne de l'élément d'étanchéité 2 par un orifice 214 et est directement en contact avec le couvercle 1 au niveau d'un orifice de sortie 12. L'embout de sortie du flux gazeux 32 est avantageusement prolongé par un tronçon rectiligne vertical 321, qui traverse l'orifice 214 de l'entretoise interne et l'orifice de sortie 12 du couvercle. L'étanchéité entre l'orifice 12 et le tronçon rectiligne 321 de l'embout 32 peut être assurée grâce à la présence d'un joint torique par exemple.

FC3 : Le couvercle 1 est relié à l'élément d'étanchéité 2 principalement grâce à une gorge 221 portée par un joint périphérique 22 de l'élément d'étanchéité 2. Cette gorge 221 assure ainsi à la fois l'étanchéité et le positionnement de l'élément d'étanchéité 2 sur le couvercle 1. Dans le présent exemple de réalisation, le réservoir présente une forme courbe ce qui assure que le joint périphérique 22 ne peut être positionné que d'une seule façon. La coopération entre le couvercle 1 et l'élément d'étanchéité 2 permet aussi de former un circuit étanche délimité par une cloison 14 du couvercle 1 qui vient s'enfoncer dans une gorge 211 formée sur l'entretoise interne 21. Il est en de même avec une cloison 15 qui vient s'enfoncer dans une gorge 213 afin de renforcer l'étanchéité autour de l'embout de sortie 32. Le détrompage est une nouvelle fois assuré par le fait que les gorge 211 et 213 n'ont pas la même forme, mais sont de forme complémentaire à celle des parois 14 et 15.

FC4 : Chacun des embouts (31, 32) est de préférence relié à l'élément d'étanchéité 2 par emmanchement. L'embout d'arrivée de flux gazeux 31 traverse l'orifice 212 tandis que l'embout de sortie du flux gazeux 32 traverse un orifice 214. Le détrompage est notamment assuré par le fait que les embouts (31, 32) ne forment qu'une pièce unique 3 qu'il n'est possible d'emmancher dans les orifices (212, 214) que d'une seule façon. De plus, une fois les embouts (31, 32) assemblés à l'élément d'étanchéité 2, il n'est possible de les fixer au couvercle 1 que d'une seule façon de par la position des éléments (34, 35, 36). L'utilisation d'un matériau souple, type polymère, est donc avantageux pour assurer étanchéité et maintien des embouts. Par ailleurs, il est alors préférable que la bride de liaison 33 vienne se positionner sous l'entretoise interne 21 lorsque le couvercle 1, l'élément d'étanchéité 2 et la pièce d'embout 3 sont assemblés de sorte que la pièce d'embout 3 ne pèse pas sur l'entretoise interne 21 et puisse aussi lui servir de support.

FC5 : Le bac 4 est principalement composé d'un fond 42, de préférence métallique, et d'une paroi latérale 41, qui relie le bac à l'élément d'étanchéité 2 grâce à une gorge 222. Ainsi, le joint périphérique 22 de l'élément d'étanchéité 2 est en compression entre le couvercle 1 et le bac 4 lorsque le réservoir est assemblé.

FC6 : La pièce d'embout 3 est avantageusement en contact avec le bac 4. La butée 36 de la bride de liaison 33 de la pièce unique d'embouts 3 est apte à venir en appui sur un élément en sailli 411 de la paroi latérale 41 du bac 4. En outre, la butée 36 vient s'insérer entre deux cales 412. La pièce d'embout 3 est alors complètement immobilisée lorsque le réservoir est assemblé. Par ailleurs, l'embout d'arrivée du flux gazeux 31 est de préférence formé d'un tronçon coudé 311 prolongé par un tronçon rectiligne 312 de sorte à ce que le flux gazeux arrive vers le centre du réservoir pour optimiser l'écoulement de gaz et que celui-ci soit suffisamment chargé en fluide avant d'atteindre l'embout de sortie 32.

FC7 : Le couvercle et le bac peuvent aussi être directement en contact, par exemple par des charnières 414 et un clip (13, 431) formé par une languette 13 portée par le couvercle et une boucle 413 portée par le bac 4. cela sécurise l'assemblage du réservoir et évite qu'il ne se décompose lorsqu'il est transporté assemblé, par exemple pour être mis en position sur un appareil apte à la recevoir.

FC8 : Enfin, le circuit patient est connecté à l'embout de sortie 32 grâce à son tronçon rectiligne 321 traversant l'orifice de sortie 12. Le positionnement du circuit patient ainsi que l'étanchéité peuvent par exemple être assurés par un emmanchement serrant, ou bien un vissage du circuit patient à l'orifice 12 ou au tronçon rectiligne 321.

## Revendications

1. Réservoir pour un appareil d'assistance respiratoire comprenant une turbine pour générer un flux gazeux, ledit réservoir comprenant un bac 4 pour contenir un fluide destiné à être entrainé par le flux gazeux, un couvercle 1, et un élément d'étanchéité 2 entre le bac 4 et le couvercle 1, **caractérisé en ce que** l'élément d'étanchéité 2 comprend un joint périphérique 22 et une entretoise interne 21 assurant le positionnement respectif d'un embout d'arrivée 31 du flux gazeux et d'un embout de sortie 32 du flux gazeux chargé en fluide.

2. Réservoir selon la revendication 1, **caractérisé en ce que** l'entretoise interne 21 de l'élément d'étanchéité 2 assure la fixation de chacun des embouts (31, 32).

3. Réservoir selon l'une quelconque des revendications 1 ou 2, **caractérisé en ce que** le couvercle 1 du réservoir comprend un orifice d'entrée 11 du flux gazeux apte à être relié à une bouche d'échappement de gaz de l'appareil, et un orifice de sortie 12 du flux gazeux chargé en fluide apte à être connecté à un conduit pour délivrer le flux gazeux chargé en fluide à un patient.

4. Réservoir selon l'une quelconque des revendications 1 à 3, **caractérisé en ce que** le couvercle 1 comprend une cloison (14, 15) apte à coopérer avec l'entretoise interne 21 de l'élément d'étanchéité 2 pour délimiter un circuit étanche.

5. Réservoir selon la revendication 4, **caractérisé en ce que** l'entretoise interne 21 présente une gorge (211, 213) apte à recevoir la cloison (14, 15) du couvercle 1 pour délimiter le circuit étanche.

6. Réservoir selon l'une quelconque des revendications 1 à 5, **caractérisé en ce que** l'embout d'arrivée 31 du flux gazeux se prolonge sous l'entretoise par un tronçon coudé 311 puis par un tronçon rectiligne 312.

7. Réservoir selon la revendication 6, **caractérisé en ce que** le tronçon rectiligne 312 de l'embout d'arrivée 31 débouche sensiblement au centre de gravité du réservoir.

8. Réservoir selon l'une quelconque des revendications 1 à 7, **caractérisé en ce que** l'embout de sortie 32 du flux gazeux se prolonge au-dessus de l'entretoise interne 21 par un tronçon rectiligne 321.

9. Réservoir selon la revendication 8, **caractérisé en ce que** le tronçon rectiligne 321 de l'embout de sortie 32 débouche à l'extérieur du couvercle 1 du réservoir.

10. Réservoir selon l'une quelconque des revendications 1 à 9, **caractérisé en ce que** l'embout d'arrivée 31 du flux gazeux est solidaire de l'embout de sortie 32 du flux gazeux grâce à une bride de liaison 33, de sorte qu'ils ne forment qu'une pièce unique 3.

11. Réservoir selon la revendication 10, **caractérisé en ce que** la bride de liaison 33 est disposée sous l'entretoise interne 21.

12. Réservoir selon l'une quelconque des revendications 10 ou 11, **caractérisé en ce que** la pièce unique 3 formée par l'embout d'arrivée 31 du flux gazeux et l'embout de sortie 32 du flux gazeux comporte un crochet de verrouillage 34 de l'assemblage de la pièce unique 3 et du couvercle 1, apte à coopérer avec un élément complémentaire porté par le couvercle 1.

13. Réservoir selon l'une quelconque des revendications 10 à 12, **caractérisé en ce que** la pièce unique 3 formée par l'embout d'arrivée 31 du flux gazeux et l'embout de sortie 32 du flux gazeux comporte une butée 35 apte à coopérer avec un élément du couvercle 1.

14. Réservoir selon l'une quelconque des revendications 10 à 13, **caractérisé en ce que** la pièce unique 3 formée par l'embout d'arrivée 31 du flux gazeux et l'embout de sortie 32 du flux gazeux comporte une butée 36 apte à venir en appui sur un élément en sailli 411 d'une paroi latérale 41 du bac 4.

15. Réservoir selon l'une quelconque des revendications 1 à 14, **caractérisé en ce que** le bac 4 du réservoir comprend un fond 42 métallique apte à être positionné sur une plaque chauffante de l'appareil.

16. Appareil d'assistance respiratoire **caractérisé en ce qu'**il comprend un réservoir selon l'une quelconque des revendications 1 à 15.

17. Appareil selon la revendication 16, **caractérisé en ce qu'**il comprend en outre un connecteur souple fixé sur la bouche d'échappement de gaz de l'appareil.

18. Utilisation d'un appareil selon l'une quelconque des revendications 16 ou 17, **caractérisé en ce que** l'on l'utilise pour le traitement de l'apnée du sommeil.
